(19) Europäisches Patentamt
European Patent Office
Office européen des brevets

(11) **EP 2 145 603 A1**

(12) **EUROPEAN PATENT APPLICATION**
published in accordance with Art. 153(4) EPC

(43) Date of publication:
**20.01.2010 Bulletin 2010/03**

(51) Int Cl.:
***A61F 2/16*** *(2006.01)*

(21) Application number: **08761598.5**

(22) Date of filing: **29.04.2008**

(86) International application number:
**PCT/ES2008/070086**

(87) International publication number:
**WO 2008/135624 (13.11.2008 Gazette 2008/46)**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **07.05.2007 ES 200701217**

(71) Applicant: **Consejo Superior De Investigaciones
Científicas
28006 Madrid (ES)**

(72) Inventors:
• **BARBERO BRIONES, Sergio**
  **E-28006 Madrid (ES)**
• **MARCOS CELESTINO, Susana**
  **E-28006 Madrid (ES)**
• **DORRONSORO DÍAZ, Carlos**
  **E-28006 Madrid (ES)**

(74) Representative: **Pons Ariño, Angel et al
Glorieta Ruben Dario 4
28010 Madrid (ES)**

(54) **ANALYTICAL DESIGN OF INTRAOCULAR LENSES**

(57) The invention relates to a method for designing intraocular lenses, comprising: the definition of a pseudoaphakic eye model; the definition of a merit function in multiple dimensions, which analytically connects the quality of the image on the retina to the optical and geometric parameters of the pseudoaphakic eye model; and the algorithm optimisation of the previous merit function using analytical and numerical methods in order to obtain one or more minimum globals which provide the optimal parameters of the intraocular lens for the pseudoaphakic eye model.

FIG 2

**Description**

**FIELD OF THE INVENTION**

The present invention relates to the field of ophthalmology, and in particular, to the design of intraocular lenses for replacing the crystalline lens in refractive eye surgery.

**STATE OF THE TECHNIQUE**

The main optical components of the eye are the crystalline lens and the cornea. These components are key in ocular refraction, the process that allows images captured by the eye to be concentrated on the retina, for subsequent transmission to the brain via the optic nerve. Various causes can lead to loss of transparency of the crystalline lens, giving rise to cataracts and the ensuing loss of vision. This defect can be treated by means of refractive surgery, a term that encompasses surgical procedures capable of correcting defects in ocular refraction. Refractive cataract surgery is based on extracting the affected crystalline lens and replacing it with an artificial lens, known as an intraocular lens (IOL). To design an intraocular lens requires selecting various parameters, such as the shape of its surfaces, its thickness and material in such a way that a suitable quality image can be obtained on the retina.

When the objective is to recover the eye's normal vision (emetropy), it is sufficient to apply monofocal designs of the IOL. However, in order to minimise spherical aberration (optical defect whereby rays of light falling on the eye are focused on different points depending on whether they fall on the periphery or on the central zone of the pupil) and other optical aberrations, other designs have to be considered, for example the use of lenses with aspheric surfaces (whose curvature is greater in the central part than in the periphery). In addition, other optical criteria have to be taken into account, in order to minimise internal reflections or to prevent aniseikonia (different size of retinal images or perceived by both eyes). The dimensions and flexibility of the IOL also have an impact on the final optical quality, since reducing the size of the corneal incision through which the lens is implanted will result in fewer corneal aberrations induced by said incision. Finally, other non-optical factors impose additional limitations on the design of IOLs. Therefore, the shape of the lens surface is key for maintaining the integrity of the crystalline lens capsule (the transparent membranous chamber in which the lens is located) and to prevent it from becoming opaque following implantation of the IOL. IOL design is therefore a problem of optimisation with boundary conditions (limitations on potential solutions).

Monofocal designs are based on the use of materials with a homogeneous refractive index and spherical or aspheric surfaces. In order to optimise the optical quality on the optical axis, the usual procedure (Lu and Smith 1990; Norrby et al. 2003) involves:

1) Evaluating the shape factor (combination of curvature radii) in order to optimise the paraxial focus, with or without additional boundary conditions;
2) Minimising the spherical aberration by giving one of the faces of the IOL asphericity.

This procedure assumes that the best quality of the on-axis image is achieved by minimising sequentially the paraxial focus (by means of modifying the curvature radii) and the spherical aberration (by means of modifying the asphericity). Upon replacing the crystalline lens with the IOL, the surgeon using standard formulae will select the paraxial power of the lens to be implanted from the set of available lenses. However, the spherical aberration of the eye interacts with the paraxial defocus, in such a way that the effective optical power may differ significantly from the paraxial power. Therefore, this interaction has been considered in order to calculate the power of the intraocular lens using ray tracing routines (Preussner et al. 2002). However, this procedure does not provide a relationship between the intended design and its parameters. In this regard, this invention proposes as the most effective procedure the design of the IOL by means of applying explicit equations that define the effective optical power according to a function of the parameters of said design applied to a pseudophakic eye model (one in which the crystalline lens has been replaced by an IOL).

Most pseudophakic eye models assume cornea models of one or two surfaces, or at most consider a multi-layer structure of the cornea and include the tear film. The present invention uses a pseudophakic eye model with the highest possible number of customised parameters. In particular, as opposed to the most common design procedures that consider IOLs to be thin lenses, the authors of the present invention propose considering IOLs to be thick lens.

Likewise, traditionally the optical quality of the IOL was evaluated using ray tracing techniques through the lenses that made it possible to introduce modifications in one or several of the design parameters in order to minimise the optical degradation (Roffman 1991; Norrby et al. 2003). Usually, in order to apply these techniques, the rays of light only had a wave length of 550 nm, corresponding to the human eye's maximum spectral sensitivity in photopic vision (full daylight). Some recent approaches (Dai; 2006) have considered polychromatic effects by introducing 7 different wave lengths as parameters of the IOL's design function, but assuming a single surface eye model. The present invention considers polychromatic effects by introducing eye model dispersion formulae of the refractive index of the different intraocular media.

Since it has been demonstrated that the equations applicable to the field of primary optical aberrations (astigmatism, spherical aberration, coma, field curvature and distortion) are useful in the design of ophthalmic lenses and in the analysis of optical quality off-axis (Lu and Smith 1990; Atchison 1991), the present invention is based on the application of these equations to the design of intraocular lenses.

Finally, we would mention that the optical quality achieved by the implantation of an IOL is limited by various biometric parameters: the positioning of the IOL in the eye (particularly in respect of the optical axis, although decentring and inclination are also important), the shape of the corneal surface after implantation of the IOL and the axial length of the eye. The present invention provides a procedure for analysing the potential effects of uncertainties regarding these biometric parameters and of manufacturing errors in the IOL that make it possible to establish a tolerance level for the design parameters: maximum acceptable variations thereof in order to maintain the optical quality of the operated eye above a particular threshold.

## DESCRIPTION OF THE INVENTION

The present invention represents a unified and complete process for designing intraocular lenses based on analytical methods derived from Gaussian optical theory and primary order optical aberrations applied to a pseudophakic eye model. The application of said methods gives rise to a figure-of-merit function, which combines the formulae of paraxial focus and spherical aberration. The optimisation of this function by means of evaluating its gradient allows us to find the optimum IOL design.

Thus, a first aspect of the invention refers to a procedure comprising the following stages:

(1) Definition of a pseudophakic eye model;
(2) Calculation of the equations providing the defocus and spherical aberration according to the parameters of said model and to the design of the intraocular lens;
(3) Configuration of a merit function based on optical quality metrics calculated analytically on the basis of the equations obtained in (2);
(4) Search for optimal designs using optimisation algorithms for the merit function developed in (3).

A second aspect of the present invention refers to the development of the pseudophakic eye model in (1) considering the intraocular lens to be a thick lens. In a preferred embodiment of the model's development, the lens has two aspheric surfaces, and therefore 4 design parameters: the curvature radii and asphericities for each one of said surfaces, since the thickness of the lens is generally conditioned by geometric considerations.

In another preferred embodiment of this aspect of the invention, the development of the pseudophakic eye model in (1) considers that the intraocular lens consists of various layers of different thicknesses, thus increasing the number of design parameters for said lens.

A third aspect of the present invention refers to the incorporation in the pseudophakic eye model developed in (1) and to the optimisation algorithms in (4) of light dispersion formulae that relate the refractive indices of the optical layers with the wave length of the light, in order to obtain designs that optimise the optical quality with polychromatic light.

A fourth aspect of this invention refers to the inclusion in the pseudophakic eye model in (1) of biometric parameters of the patient such as the shape of the cornea, intraocular distances or pupil diameter obtained using techniques available in clinical practice (videokeratoscope, optic or ultrasound biometry, pupilometer and others).

A fifth aspect of this invention refers to the inclusion in the model developed in (1) of the different layers of the cornea and the tear film obtained from histological and physiological measurements.

A sixth aspect of the invention refers to the application of the procedure to the design of multifocal IOLs by means of including in the merit function of (3), the optical quality information obtained in the retina for different distances of the target plane.

A seventh aspect of this invention refers to the inclusion in the procedure of the size of the pupil effect.

An eight aspect of the present invention refers to the generation in (4) of an analysis of tolerances, which evaluates a permissible range of errors in the different optimised design parameters in such a way that the optical quality of the design does not fall below a minimum determined threshold.

The procedure proposed in this invention improves the one normally used, based on ray tracing, due to the fact that it provides a function that explicitly relates the results of the design, in terms of image quality, to the parameters that define it. In this way, optimum designs can be found considering different boundary (limiting) conditions and making it possible to deal with the customised design of IOLs using the biometric eye parameters of a patient.

**Detailed description**

The present invention consists of a procedure for designing monofocal intraocular lenses for replacing an eye's crystalline lens, which comprises the following stages:

1) Definition of the pseudophakic eye model, consisting of:

a) an aphakic eye model (without the crystalline lens) that includes the pupil, formed by a set of concentric surfaces separating homogeneous refractive indices.
b) an intraocular lens model defined as a thick lens limited by two conic surfaces and made of any material that presents a homogeneous refractive index. Ties can be imposed between the design parameters of the intraocular lens.

In the simplest case of the pseudophakic eye model, only three surfaces are considered: one for the cornea and two for the intraocular lens. In a more general embodiment of the invention, in the pseudophakic eye model both the cornea and the intraocular lens are considered to be formed by various refractive surfaces and optical materials.

2) Defocus and spherical aberration of the eye model.
Equations are calculated for the defocus and spherical aberration of the pseudophakic eye model as a function of its parameters and of the design of the intraocular lens (curvature radii, thicknesses and refractive indices).
The paraxial power, and therefore the defocus term (Taylor term W20), of the pseudophakic eye model is obtained using the ray transfer matrix theory (Born et al. 1980). In addition it is possible to calculate the position of the paraxial image plane in respect of the retinal plane using the equation described in Smith et al., 2006.
The spherical aberration of the complete eye is calculated as the sum of the spherical aberration of all the surfaces obtained using the Schwarschild equation (Born et al. 1980). Following this procedure we obtain the spherical aberration referring to the paraxial plane (Term W40). In order to evaluate the spherical aberration in respect of the retinal plane we use the equation described in Smith et al, 2006.

3) Metrics for optimisation and configuration of the merit function.
Considering only the optical quality on-axis, the equations of W20 and W40 describe completely the geometric structure of the retinal spot (in a first approximation). The root mean square (RMS) of the wave-front is a metric used to describe geometric optical quality. Based on the obtained W20 and W40 equations it is possible to obtain an analytical equation of the RMS of the model eye using the equation described in Smith and Atchison, 1997.
In the field of optometry and ophthalmology it is more appropriate to use a metric presented in units of dioptres. In this invention, as a particular case of geometric metrics, we propose using the defocus equivalent (Me) as described in Thibos et al. 2002, in other words the defocus magnitude in dioptres necessary to produce the same magnitude of RMS on the wave-front.

4) Exploration and analysis of possible designs.
Based on the configuration of the analytical merit function we can explore the different possible designs, using different combinations of the design parameters of the intraocular lens.
The analytical merit function allows analytical boundary conditions to be introduced in order to limit the number of possible designs. Thus, for example the central thickness and thickness of the edge of the lens can be restricted, which can be useful for optimising aspects that are not purely optical, such as stability following implantation, flexibility of the lens during surgery, etc.
In synergy with the exploration stage a method is proposed for analysing the influence of changes in the eye pupil diameter on the design performances.

5) Exploitation of optimal designs: Optimisation procedure.
Following the exploration stage, the exploitation stage consists of the computerised search for optimum designs within the limited spaces. This search is based on the application of optimisation algorithms for the merit function configured in stage (3).
This invention proposes the use of algorithms based on the analytical estimation of the gradient of the merit function in respect of the different design parameters. These algorithms are more efficient and accurate than those that do not use the gradient information since they offer more complete information on the relationship between the metric and the design parameters (Fletcher 1987).

6) Analysis of tolerances and effects of the uncertainties of biometric parameters.

Once the optical design of the intraocular lens has been calculated, one mode of embodiment of the present invention comprises the analysis of tolerances of the different design parameters.

The tolerance limits are defined as the maximum allowable differences between the design values and the final manufacturing values (where deviations can occur in respect of the design values) without the optics of the lens falling below a determined threshold. The equations derived from the present procedure allow an efficient evaluation of the tolerance limits of the different parameters of the intraocular lens: curvature radii, asphericities, thickness and refractive index.

Like with the tolerance analysis, the described procedures can be used to study how errors in the measurement of the biometric parameters (such as depth of the anterior or posterior chamber) can influence the final design.

7) Complementary design procedures

7a) Design of intraocular lenses using customised eye models.

As an alternative to using the generic eye models valid for a generic patient, the present invention provides the option of designing lenses considering different individual biometric parameters. Therefore, any biometric parameter that can be measured in an individual eye can be included in the construction of a customised model.

7b) Polychromatic light

The refractive indices of the different ocular media can introduce explicitly dependency on wave length. To this effect, dispersion formulae can be introduced in the eye model such as those of Cauchy (Atchison and Smith, 2005). A polychromatic metric can be defined to calculate the analytical expressions of the defocus equivalent (Me) for different wave lengths following stage 3).

Throughout the description and the claims, the word "comprises" and its variations do not intend to exclude other technical characteristics, components, or steps. For experts in the art, other objects, benefits, and characteristics of the invention will be inferred partly from the description and partly from the practice of the invention. The examples and drawings are provided by way of illustration, and are not intended to limit the present invention.

**References**

Atchison, D. A. (1991). Design of aspheric intraocular lenses. Ophthalmic and Physiological Optics. 11 (2): 137-146.

Atchison, D. A. and G. Smith (2005). Chromatic dispersions of the ocular media of human eyes. Journal of the Optical Society of America A - Optics Image Science and Vision. 22(1): 29-37.

Barbero, S. (2006). Refractive power of a multilayer rotationally symmetric model of the human cornea and tear film. Journal of the Optical Society of America A - Optics Image Science and Vision. 23(7): 1578-1585.

Born, M., E. Wolf, et al. (1980). Principles of optics: electromagnetic theory of propagation, interference and diffraction of light. Oxford, New York.

Fletcher, R. R. (1987). Practical methods of optimization. John Wiley & Sons Ltd.

Lu, C. W. and G. Smith (1990). The aspherizing of intraocular lenses. Ophthalmic and Physiological Optics. 10(1): 54-66.

Norrby, S.; P. Artal; P.A. Piers; M. Van Der Mooren. (2003) Methods of obtaining ophthalmic lenses providing the eye with reduced aberrations. Patente US 6,609,793.

Preussner, P. R., J. Wahl, et al. (2002). Ray tracing for intraocular lens calculation. Journal of Cataract and Refractive Surgery. 28(8): 1412-1419.

Roffman, J. H. (1991). Lens design method and resulting aspheric lens. Patente US 5,050,981.

Smith, G., D. A. Atchison, et al. (2006). Effect of defocus on on-axis wave aberration of a centered optical system. Journal of the Optical Society of America A - Optics Image Science and Vision. 23(11): 2686-2689.

Smith, G. O. and D. A. Atchison (1997). The eye and visual optical instruments. Cambridge University Press.

Thibos, L. N., X. Hong, et al. (2002). Statistical variation of aberration structure and image quality in a normal population of healthy eyes. Journal of the Optical Society of America A - Optics Image Science and Vision. 19(12): 2329-2348.

Winn, B., D. Whitaker, et al. (1994). Factors affecting light-adapted pupil size in normal human subjects. Investigative Ophthalmology and Visual Science. 35(3): 1132-1137.

## BRIEF DESCRIPTION OF THE DRAWINGS

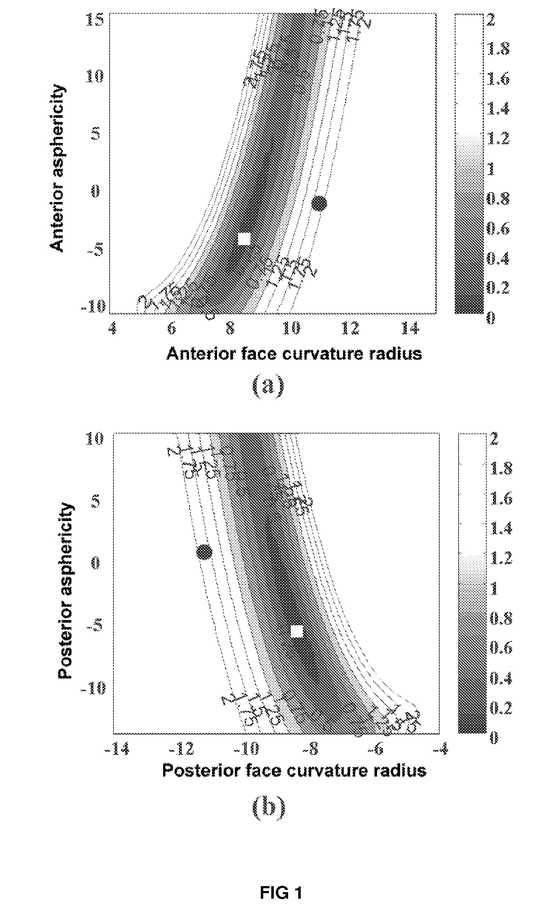

**Figure 1. Boundaries of the equivalent defocus metric for a pseudophakic eye model.**

Figure 1 represents a contour plot of the equivalent defocus metric (Me) as a function of the two curvature radii and asphericity of the anterior (a) and posterior (b) face of a generic design of a biconvex intraocular lens. The scale of greys represents Me in dioptres. The contours are represented every 0.25 dioptres. The scales of the x and y axis are different in order to provide better viewing. The results are for the customised pseudophakic eye model of table 2. The pupil radius is 2 mm.

**Figure 2. Defocus terms, spherical aberration and defocus equivalent in a pseudophakic eye model.**

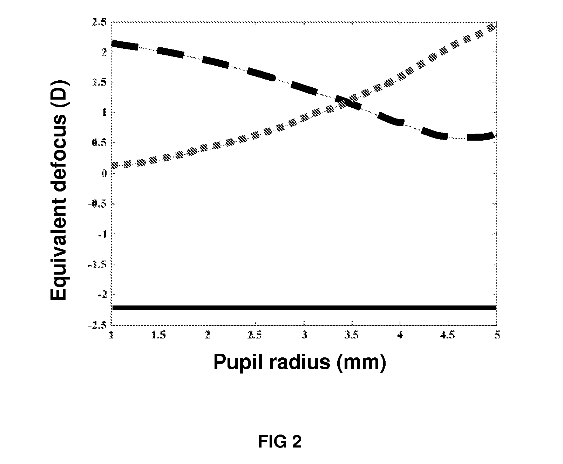

Figure 2 shows the defocus term (W20, continuous line), the spherical aberration (W40, dotted line) and the defocus equivalent in dioptres (dashed line), according to the pupil radius (mm), using the customised pseudophakic eye model of table 2.

## EXAMPLES OF EMBODIMENTS OF THE INVENTION

The following examples of embodiments of the invention are provided by way of illustration and are not intended to limit the present invention in any way.

## EXAMPLE 1. Evaluation of a commercial lens in a customised pseudophakic eye model.

In order to develop the pseudophakic eye model, a cornea model based on three layers was used: tear film, epithelium and stroma. In order to simplify the analytical expressions, a homogeneous refractive index was assumed instead of a stroma layer of index gradient. In this example of embodiment, both monochromatic and polychromatic light was used. In the case of polychromatic radiation, the variation of the refractive indices with the wave length of the different ocular media was modelled using the Cauchy dispersion formulae proposed in Atchison and Smith, 2005. For the tear film, the same dispersion was used as in the aqueous humour and for the epithelium and the stroma the dispersion equation proposed for the cornea was used. Similarly, a constant pupil with a 2 mm radius was considered which represents an average value for a normal range of luminances and ages around 70, said age being typical of eyes after cataract surgery (Winn et al. 1994). Table 1 summarises the parameters of the pseudophakic eye model used.
The parameters that can be customised for a determined eye (and that are variable between patients) are denoted by the letter C. To evaluate a commercial intraocular lens implanted in a determined eye, said parameters were customised with the data obtained from biometric measurements and from the design of the evaluated lens. The specific parameters for this model of pseudophakic eye customised to a determined eye and lens are shown in table 2, and an example of the way of obtaining them is described below.
The depth of the anterior chamber, distance between the vertex of the posterior surface of the cornea and the vertex of the anterior surface of the intraocular lens were determined using a slit lamp, while the axial length of the eye was determined by partial coherence interferometry using an IOL Master (Zeiss). The shape of the anterior surface of the cornea (surface of separation between the air and the tear film) was obtained from a corneal topography measurement using an Atlas Humphrey Instruments device (Zeiss) adjusted to a conical shape in order to obtain the radius and asphericity of the tear film. Based on these data and following a procedure described in Barbero (2006), the curvature radii and asphericities were obtained of the rest of the surfaces of the corneal layers.
The design parameters of the lens (22 D Tecnis Z9000 IOL) were taken from Norrby et al. 2003.
As a metric the defocus equivalent was used with monochromatic radiation (555 nm), obtaining as a result, for this particular lens and in this specific eye, a value of 1.2 Dioptres. Since this is more than 0.25 D, it was considered that this value was significant and that the final optical quality was poor.

**EXAMPLE 2. Design of new intraocular lenses in a customised pseudophakic eye model.**

For the same model developed for example 2 the commercial intraocular lens was replaced with another generic lens of the same material, described by two conical surfaces of variable parameters, and the exploratory analysis was carried out of the possible lens designs. The value of the optical quality metric for the different combinations of design parameters is represented as a multidimensional surface of solutions (each solution being a given design).

The multidimensional surfaces are shown as partial two-dimensional graphs that represent cuts in the solutions space when two design parameters are established.

In this particular embodiment of the invention figure 1 shows the value of the defocus equivalent according to the curvature radius and asphericity (for a biconvex lens), both for the anterior surface (a) as well as for the posterior surface (b). In other embodiments the exploratory analysis serves to study the dependence of the final quality on other parameters of the lens or of the model eye itself, such as pupil diameter, depth of the anterior chamber, or axial length.

Figure 1 shows extensive regions (combinations of parameters) where the metric obtained reached a value of less than 0.25 D (zones in black), indicating that the design had optimum performances. The black circle of figure 1 shows where the solution represented by the commercial lens implanted in that eye is situated, which is very distant from the zone of optimum performance (metric below 0.25 D), indicating that there are designs for the lens that in this eye provide a significantly better defocus equivalent than said commercial lens and therefore, an improved optical quality.

For the optimisation of the design, the gradient of the multidimensional analytical surface was calculated, as described in the detailed description of this invention. In this example of embodiment, due to the fact that the relation between the defocus equivalent and the radii and asphericities was clearly nonlinear, both first and second derivatives were considered and the Quasi-Newton optimisation algorithm was used (Fletcher 1987), which establishes the search direction in an iterative process and provides good results in few iterations.

Table 3 shows (row IOLC1) the optimum design for this customised eye and monochromatic radiation (555 nm). This optimisation result is also shown graphically in figure 1 as a white square.

Table 3 also shows the global optimum when polychromatic light is considered (IOLP1). For this calculation, dispersion formulae of the refractive index of table 2 were considered and a polychromatic optical quality metric (Mew = polychromatic defocus equivalent) defined by the following formula:

$$Mew = Me(420.7) + 0.5 \times (Me(530.3) + Me(558.9))$$

based on the capture of a luminous signal by the three types of photoreceptors existing in the retina, each one of which shows a spectral sensitivity: short cones to 422.7 nm, medium to 530.3 nm and long to 558.9 nm. This polychromatic metric is just one example of the many that can be defined. For optimisation, the starting point was the best monochromatic result (Table 4, IOLC1), and slightly different design parameters were obtained, representing the best point of compromise between the results for the different evaluated wave lengths.

**EXAMPLE 3. Analysis of tolerances applied to the design of new intraocular lenses in a customised pseudophakic eye model.**

Variations were analysed in the spherical aberration and defocus equivalent with the pupil diameter using the customised pseudophakic eye model of table 2. Figure 2 shows how a customised intraocular lens for a patient has a determined pupil size that optimises the metric and therefore its performances. In this example the optimum pupil radius is 4.8 mm (Me=0.68 D). A change of 0.25 D in the defocus equivalent was assumed to be acceptable, and this error was extended to the parameters to find their maximum permissible variation. Table 4 shows the tolerance limits that were obtained for the curvature radii, asphericities, the thickness and refractive index.

**Table 1. Parameters of a generic pseudophakic eye model.**

| | Interface | | | | | |
|---|---|---|---|---|---|---|
| | I1 | I2 | I3 | I4 | I5 | I6 |
| Radius (mm) | C | C | C | 6.4 | C | C |
| Asphericity | C | C | C | -0.38 | C | C |

(continued)

| Mean | | | | | | |
|---|---|---|---|---|---|---|
| | Tear | Epithelium | Stroma | Aqueous | ILO | Vitreous |
| Central thickness (mm) | 0.004 | 0.0537 | 0.473 | C | C | C |
| Refractive index | $n(\lambda)_a$ | $n(\lambda)_c$ | $n(\lambda)_c$ | $n(\lambda)_c$ | C | $n(\lambda)_v$ |

C represents a customisable parameter. The interfaces are I1: air-tear; I2: tear epithelium; I3: epithelium-stroma; I4: stroma-aqueous; I5: aqueous-intraocular lens; and I6: intraocular lens-vitreous. The dispersion formulae of the refractive index for the aqueous, vitreous and corneal media are $n(\lambda)_a$, $n(\lambda)_v$ and $n(\lambda)_c$, respectively (Atchison and Smith, 2005).

### Table 2. Parameters of a customised pseudophakic eye model.

| Interface | | | | | | |
|---|---|---|---|---|---|---|
| | I1 | I2 | I3 | I4 | I5 | I6 |
| Radius (mm) | 7.79 | 7.79 | 7.56 | 6.4 | 11.043 | -11.043 |
| Asphericity | -0.49 | -0.49 | -1.9 | -0.38 | -1.036 | 0 |
| Mean | | | | | | |
| | Tear | Epithelium | Stroma | Aqueous | ILO | Vitreous |
| Central thickness (mm) | 0.004 | 0.0537 | 0.473 | 4.29 | 1.164 | 17 |
| Refractive index | $n(\lambda)_a$ | $n(\lambda)_c$ | $n(\lambda)_c$ | $n(\lambda)_a$ | $n(\lambda)_s$ | $n(\lambda)_v$ |

The interfaces are I1: air-tear; I2: tear-epithelium; I3: epithelium-stroma; I4: stroma-aqueous; I5: aqueous-intraocular lens; and I6: intraocular lens-vitreous. The dispersion formulae of the refractive index for aqueous, vitreous, corneal media and silicone are $n(\lambda)_a$, $n(\lambda)_v$, $n(\lambda)_c$ and $n(\lambda)_s$, respectively (Atchison and Smith, 2005).

### Table 3: Design specifications and optical performances of intraocular lenses obtained with monochromatic and polychromatic radiation.

| Optimisation algorithm | Ra (mm) | Qa | Rp (mm) | Qp | Me (D) |
|---|---|---|---|---|---|
| Cuasi-Newton | 8.97 | -2.82 | -10.48 | -2.25 | $4 \times 10^{-7}$ |
| Nelder-Mead | 5.92 | -0.82 | -32 | 0.91 | $2.4 \times 10^{-4}$ |

| Optimisation algorithm | Number of iterations | Number of functions |
|---|---|---|
| Cuasi-Newton | 30 | 226 |
| Nelder-Mead | 480 | 800 |

Ra and Rp represent the anterior and posterior curvature radii of the intraocular lens; Qa and Qp represent the anterior and posterior asphericities of the intraocular lens. Me represents the equivalent defocus (in dioptres, D) of the intraocular lens in the model of table 3. The number of required iterations to find the optimised algorithm is shown and the number of functions evaluated during said optimisation.

### Table 4: Tolerance limits of the pseudophakic eye model of table 2 for the design parameters of the intraocular lens.

| Design IOL | Ra (mm) | Qa | Rp (mm) | Qp | Me (D 555 nm) | W20 (D) | W40 (D) |
|---|---|---|---|---|---|---|---|
| IOL A1 | 9.05 | 0 | -11.04 | 0 | 0.12 | -0.48 | 0.49 |

(continued)

| Design IOL | Ra (mm) | Qa | Rp (mm) | Qp | Me (D 555 nm) | W20 (D) | W40 (D) |
|---|---|---|---|---|---|---|---|
| IOL A2 | 8.63 | 0 | -11.04 | 0 | 0.5 | 0 | 0.5 |
| IOL B1 | 9.05 | -0.27 | -11.04 | 0 | 0.12 | -0.47 | 0.46 |
| IOL B2 | 9.05 | 0 | -11.04 | -0.59 | 0.12 | -0.47 | 0.46 |
| IOL B3 | 8.63 | -3.38 | -11.04 | 0 | 0.0007 | 0.0030 | -0.0029 |
| IOL B4 | 8.63 | -3.37 | -11.04 | 0 | 0.003 | 0.003 | 0 |
| IOL B5 | 8.63 | 0 | -11.04 | -8.74 | 0.0007 | 0.003 | -0.003 |
| IOL B6 | 8.63 | 0 | -11.04 | -8.69 | 0.003 | 0.003 | 0 |
| IOL C1 | 8.63 | -3.37 | -11.04 | 0 | $0.1 \times 10^{-6}$ | $-0.5 \times 10^{-6}$ | $0.5 \times 10^{-6}$ |
| IOL P1 | 8.71 | -3.45 | -11.04 | 0 | 0.09 | -0.09 | $-4 \times 10^{-4}$ |

Ra and Rp represent the anterior and posterior curvature radii of the intraocular lens; Qa and Qp represent the anterior and posterior asphericities of the intraocular lens. W20, W40 and Me represent the equivalent defocus (in dioptres, D) due to the defocus terms, spherical aberration and combination of the two, respectively. The optimisation procedure of the merit function gave rise to the different designs of intraocular lenses (series IOL A, IOL B, IOL C and IOL P).

**Claims**

1. Procedure for the design of intraocular lenses, **characterised in that** it comprises the following stages:

    a) Definition of a pseudophakic eye model, which consists of:

        i. an aphakic eye model that comprises various optical surfaces, refractive indices and thicknesses that describe the optical elements of the eye without the crystalline lens, and
        ii. an intraocular lens model that comprises a succession of optical surfaces and separated by layers of material described by their thickness and refractive index,

    b) Definition of a merit function in various dimensions that relates analytically the quality of the image in the retina with the optical and geometric parameters of the pseudophakic eye model, wherein the parameters of the aphakic eye model are established as fixed terms and the parameters of the intraocular lens are considered variable terms, and
    c) The algorithmic optimisation of the merit function above by analytical and numerical means in order to find one or several global minimums from which the optimum parameters of the intraocular lens can be obtained for the established pseudophakic eye model.

2. Procedure according to claim 1, **characterised in that** the aphakic eye model is established by the inclusion of the mean biometric parameters of the eyes of a population of patients that may be grouped according to different indices related to said biometric parameters, such as age or refractive error.

3. Procedure according to claim 1 **characterised in that** the aphakic eye model is developed including some of the biometric parameters of a given patient, such as corneal geometry, intraocular distances and pupil diameter.

4. Procedure according to claim 1 **characterised in that** the generation of a merit function comprises the following stages:

    a) Evaluation of defocus in the paraxial plane given by the eye model, using the matrix formalism of paraxial ray transfer,
    b) Evaluation of the spherical aberration in the paraxial plane using the third order aberration theory,
    c) Application of an analytical corrective factor, using a plane change equation, for a better estimation of both spherical aberration and defocus in the retina plane,
    d) Obtaining the wave aberration variance function based on the previous equations and, as a final merit function, the equivalent in dioptres corresponding to said variance.

5. Procedure according to claim 1 **characterised in that** the optimisation of the merit function is carried out by analytically obtaining the partial derivatives of the multidimensional merit function and obtaining a global minimum (or several local minimums) across the gradients thus calculated.

6. Procedure according to any of the preceding claims **characterised in that** both in the pseudophakic eye model as well as in the calculation and optimisation of the multidimensional merit function refractive indices are included that depend on the wave length (dispersion formulae).

7. Procedure according to any of the preceding claims **characterised in that** the multidimensional merit function incorporates optical quality parameters for different distances of the target plane, it being possible to extend said procedure to the design of multifocal intraocular lenses.

8. Procedure according to any of the preceding claims **characterised in that** in the aphakic eye model the cornea is considered to be an element with multiple optical layers, defined by its border surfaces, thicknesses and refractive indices.

9. Procedure according to claim 4 **characterised in that** the generation of the merit function can be carried out using the optical metrics calculated analytically on the basis of the spherical aberration and the defocus in the retina calculated previously.

10. Procedure according to claim 9 **characterised in that** the metrics used is visual metrics that include the expected response of the visual system to the retinal optical stimulus.

11. Procedure according to claim 9 **characterised in that** the metrics used includes the estimation of the diffractives on the image plane.

12. Use of the procedures of the preceding claims for the analysis of tolerances for the lens in manufacturing and quality control processes.

FIG 1

FIG 2

# INTERNATIONAL SEARCH REPORT

| International application No. |
| --- |
| PCT/ ES 2008/070086 |

**A. CLASSIFICATION OF SUBJECT MATTER**

*A61F 2/16* (2006.01)

According to International Patent Classification (IPC) or to both national classification and IPC

**B. FIELDS SEARCHED**

Minimum documentation searched (classification system followed by classification symbols)

Documentation searched other than minimum documentation to the extent that such documents are included in the fields searched

Electronic data base consulted during the international search (name of data base and, where practicable, search terms used)

INVENES,EPODOC

**C. DOCUMENTS CONSIDERED TO BE  RELEVANT**

| Category* | Citation of document, with indication, where appropriate, of the relevant passages | Relevant to  claim No. |
| --- | --- | --- |
| A | WO 2006053216 A1 (ADVANCED MEDICAL OPTICS,INC;NORRBY, SVERKER) 18.05.2006, the whole document. | 1-3 |
| A | (JUAN TABERNERO et al (PREDICTING THE OPTICAL PERFORMANCE OF EYES IMPLANTED WITH IOLS TO CORRECT SPHERICAL ABERRATION)INVESTIGATIVEE OPHTHALMOLOGY & VISUAL SCIENCE, OCTOBER 2006, VOL47 Nº 10) the whole document. | 1-3 |
| A | ES 2245968 A1 (BAUSCH & LOMB, INC. 10.09.2003) the whole document. | 1-3 |

☐ Further documents are listed in the continuation of Box C.　　　☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | | |
| | | "&" | document member of the same patent family |

| Date of the actual completion of the international search | Date of mailing of the international search report |
| --- | --- |
| 26.August.2008　　　(26.08.2008) | (28-08-2008) |
| Name and mailing address of the ISA/ O.E.P.M. <br><br> Paseo de la Castellana, 75 28071 Madrid, España. <br> Facsimile No.　34 91 3495304 | Authorized officer <br><br> G. Foncillas Garrido <br><br> Telephone No. +34 91 349 32 82 |

Form PCT/ISA/210 (second sheet) (July 2008)

## INTERNATIONAL SEARCH REPORT

Information on patent family members

| | International application No. |
|---|---|
| | PCT/ ES 2008/070086 |

| Patent document cited in the search report | Publication date | Patent family member(s) | Publication date |
|---|---|---|---|
| EP 1341486 | 10.09.2003 | US 2002072796 A | 13.06.2002 |
| | | CA 2430005 AC | 20.06.2002 |
| | | WO 0247584 A | 20.06.2002 |
| | | AU 1994802 A | 24.06.2002 |
| | | EP 1341486 AB | 10.09.2003 |
| | | EP 20010270289 | 28.11.2001 |
| | | AR 031795 A | 01.10.2003 |
| | | US 2003195622 A | 16.10.2003 |
| | | US 6755859 B | 29.06.2004 |
| | | BR 0116518 A | 13.01.2004 |
| | | CN 1479598 A | 03.03.2004 |
| | | JP 2004515309 T | 27.05.2004 |
| | | ZA 200304066 A | 26.08.2004 |
| | | ES 2245968 T | 01.02.2006 |
| | | WO 0247584 | 20.06.2002 |
| | | WO 0187188 [A | 22.11.2001 |
| | | DE 19925636 [A | 07.12.2000 |
| | | EP 0962196 [Y | 08.12.1999 |
| | | US 6152959 [Y | 28.11.2000 |
| | | WO 9220302 [Y | 26.11.1992 |
| | | EP 0492126 [Y | 01.07.1992 |
| | | US 4198980 [A | 22.04.1980 |
| | | US 5873879 /R | 23.02.1999 |
| | | US 5860986 /R | 19.01.1999 |
| | | US 5810834 /R | 22.09.1998 |
| | | US 5326506 /R | 05.07.1994 |
| | | US 5217491 /R | 08.06.1993 |
| WO 2006053216 | 18.05.2006 | CA 2587432 A | 18.05.2006 |
| | | AU 2005304382 A | 18.05.2006 |
| | | WO 2006053216 A | 18.05.2006 |
| | | EP 1827313 A | 05.09.2007 |
| | | EP 20050851545 | 11.11.2005 |
| | | US 2007260157 A | 08.11.2007 |
| | | US 2004088050 [A | 06.05.2004 |
| | | WO 03022137 [A | 20.03.2003 |
| | | WO 02088830 [A | 07.11.2002 |
| | | US 5968095 [A | 19.10.1999 |

Form PCT/ISA/210 (patent family annex) (July 2008)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6609793 B

- US 5050981 A

**Non-patent literature cited in the description**

- **Atchison, D. A.** Design of aspheric intraocular lenses. *Ophthalmic and Physiological Optics,* 1991, vol. 11 (2), 137-146
- **Atchison, D. A. ; G. Smith.** Chromatic dispersions of the ocular media of human eyes. *Journal of the Optical Society of America A - Optics Image Science and Vision,* 2005, vol. 22 (1), 29-37
- **Barbero, S.** Refractive power of a multilayer rotationally symmetric model of the human cornea and tear film. *Journal of the Optical Society of America A - Optics Image Science and Vision,* 2006, vol. 23 (7), 1578-1585
- **Born, M. ; E. Wolf et al.** *Principles of optics: electromagnetic theory of propagation, interference and diffraction of light,* 1980
- **Fletcher, R. R.** Practical methods of optimization. John Wiley & Sons Ltd, 1987
- **Lu, C. W. ; G. Smith.** The aspherizing of intraocular lenses. *Ophthalmic and Physiological Optics,* 1990, vol. 10 (1), 54-66

- **Preussner, P. R. ; J. Wahl et al.** Ray tracing for intraocular lens calculation. *Journal of Cataract and Refractive Surgery,* 2002, vol. 28 (8), 1412-1419
- **Smith, G. ; D. A. Atchison et al.** Effect of defocus on on-axis wave aberration of a centered optical system. *Journal of the Optical Society of America A - Optics Image Science and Vision,* 2006, vol. 23 (11), 2686-2689
- **Smith, G. O. ; D. A. Atchison.** The eye and visual optical instruments. Cambridge University Press, 1997
- **Thibos, L. N. ; X. Hong et al.** Statistical variation of aberration structure and image quality in a normal population of healthy eyes. *Journal of the Optical Society of America A - Optics Image Science and Vision,* 2002, vol. 19 (12), 2329-2348
- **Winn, B. ; D. Whitaker et al.** Factors affecting light-adapted pupil size in normal human subjects. *Investigative Ophthalmology and Visual Science,* 1994, vol. 35 (3), 1132-1137